(19) Europäisches Patentamt

European Patent Office

Office européen des brevets

(11) **EP 4 124 854 A1**

(12) **EUROPEAN PATENT APPLICATION**

(43) Date of publication:
**01.02.2023 Bulletin 2023/05**

(51) International Patent Classification (IPC):
***G01N 27/327*** (2006.01)

(21) Application number: **22152850.8**

(22) Date of filing: **24.01.2022**

(52) Cooperative Patent Classification (CPC):
**G01N 27/3274**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2021 TW 110127664**

(71) Applicant: **Apex Biotechnology Corp.**
**Hsinchu (TW)**

(72) Inventors:
• **YANG, Chen-Yu**
**351 Toufen City, Miaoli County (TW)**
• **TSENG, Yu-Han**
**300 Hsinchu City (TW)**

(74) Representative: **Becker Kurig & Partner**
**Patentanwälte mbB**
**Bavariastraße 7**
**80336 München (DE)**

(54) **ELECTROCHEMICAL DETECTION SYSTEM, MEASURING INSTRUMENT AND ELECTROCHEMICAL DETECTION METHOD**

(57)     An electrochemical detection system is configured to detect a sample. The electrochemical detection system includes an electrochemical test strip and a measuring instrument. The electrochemical test strip includes a main body and at least one electrode group. The measuring instrument is electrically connected to the at least one electrode group. The measuring instrument is adapted to determine whether a flow field condition of the sample is normal via the at least one electrode group.

FIG. 1A

**Description**

BACKGROUND OF THE INVENTION

Field of the Invention

The invention relates to an electrochemical detection system, a measuring instrument, and an electrochemical detection method, and particularly relates to an electrochemical detection system, a measuring instrument, and an electrochemical detection method that may improve detection accuracy.

Description of Related Art

In medical or biochemical testing, the use of an electrochemical test strip is already a common technique, such as to measure the sample concentration. When sample concentration is measured, after the sample is reacted with the reaction layer in the electrochemical test strip, the electrode in the electrochemical test strip may be configured to provide a reaction voltage and receive a signal. Then, sample concentration is calculated based on the Cottrell equation.

However, in the process of using an electrochemical test strip to detect a sample, the mixing reaction time between the sample and the reaction layer is usually abnormal due to unexpected flow fields such as secondary sample feeding, abnormal flow channels, or extreme samples. As a result, the measuring instrument misses the best signal acquisition time and produces a result with deviation, and the result of the deviation signal may still fall within the preset threshold value interpreted by the instrument. In other words, the instrument cannot exclude the result with deviation signal via the setting of the threshold value, so that the measurement result of the sample concentration will have a significant error. Therefore, if the reaction signals under these abnormal conditions may not be effectively distinguished or eliminated, the measurement result will be erroneous, thereby reducing the detection accuracy of the electrochemical test strip.

SUMMARY OF THE INVENTION

Accordingly, the invention provides an electrochemical detection system, a measuring instrument, and an electrochemical detection method that may have the effect of improving detection accuracy.

The electrochemical detection system of the invention may be configured to detect a sample. The electrochemical detection system includes an electrochemical test strip and a measuring instrument. The electrochemical test strip includes a main body and at least one electrode group. The measuring instrument is electrically connected to the at least one electrode group. The measuring instrument is adapted to determine whether a flow field condition of the sample is normal via the at least one electrode group.

In an embodiment of the invention, the measuring instrument measures a first characteristic and a second characteristic of at least one of the sample and the electrochemical test strip via the at least one electrode group, and calculates a ratio of the first characteristic to the second characteristic to determine whether a flow field condition of the sample is normal. When the ratio is within a predetermined range, the measuring instrument determines that the flow field condition is normal and displays a result of an analysis of the sample.

In an embodiment of the invention, the first characteristic or the second characteristic is a fluid characteristic.

In an embodiment of the invention, the fluid characteristic includes a flow velocity, a temperature, a viscosity, and a density of the sample.

In an embodiment of the invention, the first characteristic or the second characteristic is a flow channel characteristic.

In an embodiment of the invention, the flow channel characteristic includes a contact angle, a charging current, a diameter of a reaction area of the sample, a volume flow rate of the sample, and a cross-sectional area of the reaction area.

In an embodiment of the invention, there is a low equivalence between the first characteristic and the second characteristic.

In an embodiment of the invention, the flow field condition includes an abnormal flow channel and an abnormal sampling.

The measuring instrument of the invention may be configured to detect a sample and includes a connector, a microcontroller, a power module, and a display. The microcontroller is electrically connected to at least one electrode group via the connector. The power module is electrically connected to the microcontroller. The display is electrically connected to the microcontroller. The microcontroller is adapted to measure a first characteristic and a second characteristic of the sample via the at least one electrode group, and analyze a relationship between the first characteristic and the second characteristic and a flow field.

An electrochemical detection method of the invention may be configured to detect a sample and includes the following steps. The sample is injected into an electrochemical test strip. Whether a flow field condition of the sample is normal is determined via a measuring instrument. In particular, the step of determining whether the flow field condition of the sample is normal includes: measuring a first characteristic and a second characteristic of at least one of the sample and the electrochemical test strip via at least one electrode group of the electrochemical test strip; calculating a ratio of the first characteristic to the second characteristic. When the ratio is within a predetermined range, the measuring instrument determines that the flow field condition is normal and displays a result of an analysis of the sample.

In an embodiment of the invention, the first characteristic relates to an ion diffusion and/or a migration ability of the sample.

In an embodiment of the invention, the first characteristic includes a flow velocity, a temperature, a viscosity, and a density of the sample.

In an embodiment of the invention, the second characteristic is related to a sample and electrode interface characteristic.

In an embodiment of the invention, the second characteristic includes a contact angle, a charging current, a diameter of a reaction area of the sample, a volume flow rate of the sample, and a cross-sectional area of the reaction area.

Based on the above, in the electrochemical detection system and the electrochemical detection method of an embodiment of the invention, since the measuring instrument may be electrically connected to at least one electrode group of the electrochemical test strip, and whether the flow field condition of the sample is normal is determined via the at least one electrode group, the electrochemical detection system and the electrochemical detection method of the present embodiment may avoid errors, or may have the effect of improving detection accuracy.

In order to make the aforementioned features and advantages of the disclosure more comprehensible, embodiments accompanied with figures are described in detail below.

BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

FIG. 1A is a schematic block diagram of an electrochemical detection system of an embodiment of the invention.
FIG. 1B is an exploded view of the structure of the electrochemical test strip of FIG. 1A.
FIG. 1C is a schematic top view of area A in the electrochemical test strip of FIG. 1B.
FIG. 2 is a flowchart of an electrochemical detection method of an embodiment of the invention.
FIG. 3 shows the use of an electrochemical detection system of an embodiment of the invention to measure the viscosity of blood.
FIG. 4 shows the use of an electrochemical detection system of an embodiment of the invention to identify abnormal conditions of secondary sample feeding.
FIG. 5 is a graph showing the relationship between contact angle and resistance of a detected sample to an electrochemical test strip.
FIG. 6 is a graph showing the relationship between contact angle and viscosity of a detected sample to an electrochemical test strip.
FIG. 7A shows measurement deviation values after measuring blood sugar concentration using a general electrochemical detection method.
FIG. 7B shows measurement deviation values after measuring blood sugar concentration using the electrochemical detection method of an embodiment of the invention.

DESCRIPTION OF THE EMBODIMENTS

The following "liquid intrinsic characteristic", "liquid electrical characteristic" and "intrinsic electrical characteristic" all correspond to the above "fluid characteristic".

The following "sample and electrode interface characteristic", "flow channel wall interface characteristic", "flow channel interface characteristic", and "flow channel interface characteristic" all correspond to the above "flow channel characteristic".

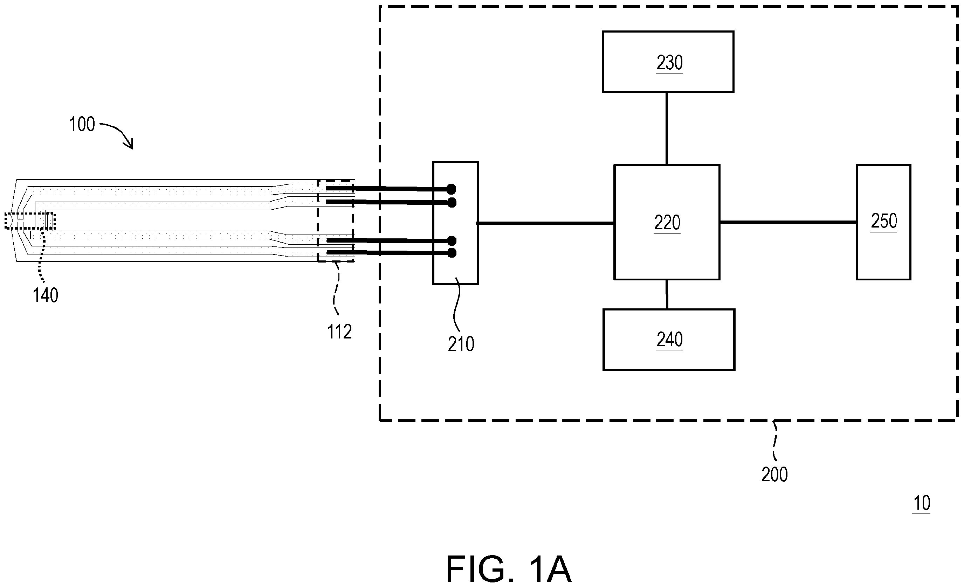

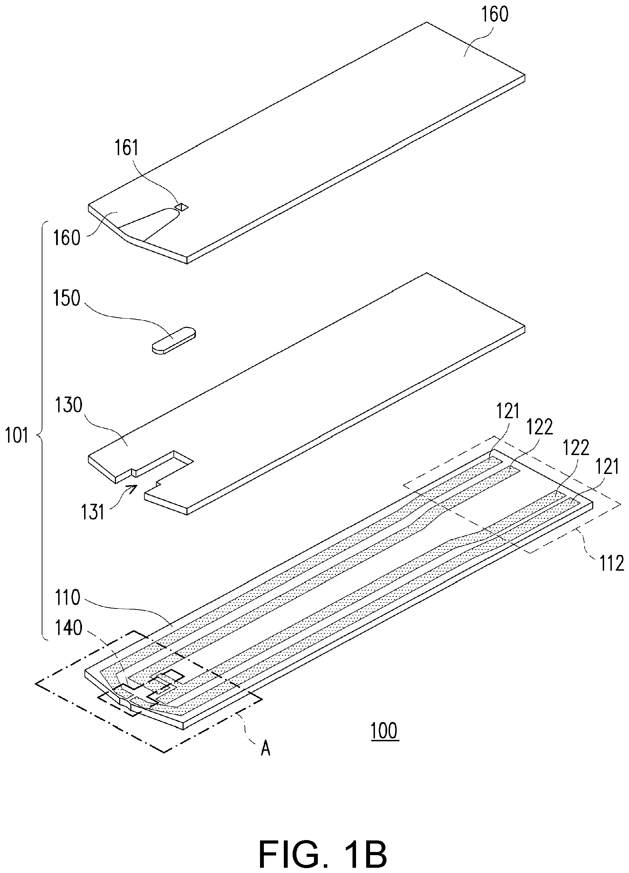

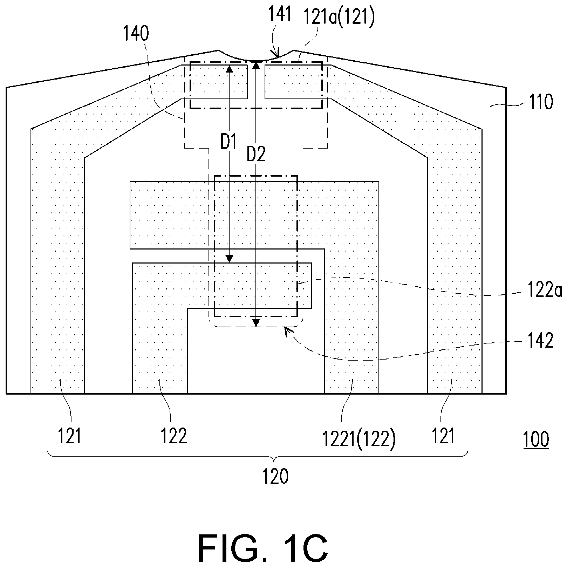

FIG. 1A is a schematic block diagram of an electrochemical detection system of an embodiment of the invention. FIG. 1B is an exploded view of the structure of the electrochemical test strip of FIG. 1A. FIG. 1C is a schematic top view of area A in the electrochemical test strip of FIG. 1B. For clarity of the figures and convenience of description, FIG. 1A and FIG. 1C omit several elements in the electrochemical test strip.

Referring to FIG. 1A to FIG. 1C, an electrochemical detection system 10 of the present embodiment may be configured to detect a sample. The electrochemical detection system 10 includes an electrochemical test strip 100 and a measuring instrument 200. The electrochemical test strip 100 includes a main body 101 and at least one electrode group 121 and 122. In particular, the main body 101 includes an insulating substrate 110, a first insulating spacer 130, a reaction area 140, a reaction layer 150, and a second insulating spacer 160. The electrode groups 121 and 122 are disposed on the insulating substrate 110. The first insulating spacer 130 and the second insulating spacer 160 are sequentially stacked on the insulating substrate 110. The electrode groups 121 and 122 are partially located at a concave structure 131 of the first insulating spacer 130. The reaction layer 150 is disposed on the insulating substrate 110 and located in the concave structure 131 of the first insulating spacer 130. The reaction area 140 may be defined as an area surrounded by the concave structure 131.

In the present embodiment, the measuring instrument 200 includes a connector 210 for external connection, a microcontroller (MCU) 220, a power module 230, a temperature sensor 240, and a display 250. The connector 210 of the measuring instrument 200 may be electrically connected to a connection area 112 of the electrochemical test strip 100. The microcontroller 220 is electrically connected to the at least one electrode group 121 and 122 via the connector 210. The power module 230, the temperature sensor 240, and the display 250 are electrically connected to the microcontroller 220, respectively.

In the present embodiment, the measuring instrument 200 is adapted to measure the first characteristic and the second characteristic of the sample via the at least one electrode group 121 and 122, and analyze the relationship between the first characteristic and the second characteristic and the flow field to determine whether the flow field condition of the sample is normal. Then, when the ratio is within a predetermined range, the measuring instrument 200 determines that the flow field condition is normal and displays a result of an analysis of the sample. In particular, the first characteristic is to measure the liquid intrinsic characteristic of the such as the ion diffusion and/or migration ability of the sample, thereby analyzing a physical characteristic of the liquid sample. The physical characteristic includes, but is not limited to, viscosity, diffusion coefficient, and the like. The second characteristic is to measure the sample electrode interface characteristic, so as to analyze the flow channel wall interface characteristic. The flow channel characteristic includes, but is not limited to, contact angle, charging current, and the like. That is, the first characteristic is related to the ion diffusion and/or migration ability of the sample, and the second characteristic is related to the sample and electrode interface characteristic. It should be noted that, the present application does not limit the measurement methods of the first characteristic and the second characteristic. In an embodiment, the characteristic is an electrical signal, so the first and second characteristics are the liquid electrical characteristic and the liquid and electrode interface electrical characteristic, respectively. In another embodiment, both the first and second characteristics are the liquid electrical characteristic and the liquid and electrode interface electrical characteristic. In another embodiment, at least one of the first and second characteristics is measured by an optical method.

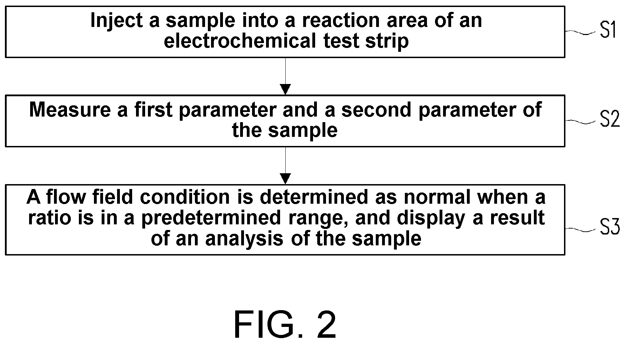

FIG. 2 is a flowchart of an electrochemical detection method of an embodiment of the invention. The electrochemical detection method of the present embodiment may be configured to detect the first characteristic and the second characteristic of the sample. In particular, the electrochemical detection method includes the following steps: step S1, a sample is injected into the reaction area 140 of the electrochemical test strip 100; step S2, the first characteristic of the sample and the second characteristic of the sample are measured; step S3, the ratio of the first characteristic to the second characteristic is calculated, so as to determine whether the flow field condition of the sample filled in the reaction area 140 is normal. When the ratio is within a predetermined range, the flow field condition is determined as normal and a result of an analysis of the sample is displayed.

Specifically, referring to all of FIG. 1A to FIG. 1C and FIG. 2, in the present embodiment, the electrochemical test strip 100 is inserted into the measuring instrument 200, and the microcontroller 220 provides a potential to the reaction area 140. When the sample is injected into the reaction area 140 of the electrochemical test strip 100 and covers the electrode circuit of the electrode group 121 so that the impedance of the electrode group 121 is changed, the microcontroller 220 may confirm that the sample has begun to flow into the reaction area 140. In the present embodiment, the microcontroller 220 may determine whether the sample is filled in the reaction area 140 via the change of the signal threshold value of the electrode group 122 (such as resistance, capacitance, voltage, current, or a combination thereof); in the present embodiment, the injection and filling conditions of the sample may be determined via the significance on the positions of the electrode group 121 and the electrode group 122, as shown in FIG. 1C. Since the electrode group 121 is closer to the sampling port (i.e., the first side 141), and the electrode group 122 is closer to the end of the reaction area 140 (i.e., the second side 142), the sample may be determined as injected into the starting point using the electrode group 121, and the sample may be determined as filled using the electrode group 122.

In the present embodiment, the microcontroller 220 detects the first characteristic and the second characteristic of the sample and starts to analyze the flow field condition after the sample is filled. In another embodiment, the microcontroller 220 detects the first characteristic and the second characteristic of the sample during the filling process of the sample, including, but not limited to, an electrical signal used to determine the filling; in another embodiment, the microcontroller 220 detects the first characteristic or the second characteristic of the sample during and after the sample is filled.

Under normal circumstances, the sample flows into the reaction area 140 as a one-dimensional natural flow due to capillary force. However, the sample may have an abnormal flow field condition due to abnormal flow patterns or abnormal flow channel structures. In other words, when the sample flows gradually under the action of capillary force, an unpredictable flow time difference between the sample and the tube channel boundary of the reaction area 140 occurs due to non-predetermined effects. The difference may be caused by factors such as friction, disturbance, or turbulence generated by the flowing fluid, and the abnormal flow field condition causes errors or inaccuracy in the measurement results of the sample concentration. Therefore, one of the objectives of the present embodiment is to eliminate the error of the sample concentration measurement result caused by abnormal flow field by determining the flow field condition of the sample, thereby improving detection accuracy and reliability. More specifically, in the present embodiment, by measuring the sample interface electrical characteristic and the sample and electrode interface electrical characteristic, respectively, the flow time difference caused by the sample and the flow channel to the flow field is analyzed respectively to eliminate or correct the measurement deviation caused by the abnormal flow field.

In detail, in the present embodiment, the first characteristic and the second characteristic may include two of the flow velocity, temperature, viscosity, density, and contact angle of the sample, but is not limited thereto. In the present embodiment, by performing a two-dimensional cross-analysis on at least two detected characteristics (for example, the first characteristic and the second characteristic), the flow field condition of the sample in the reaction area 140 may be known. It should be noted that the two characteristics are not limited, as long as the two characteristics do not have the same physical meaning (that is, the first characteristic and the second characteristic have low equivalence), and both of the two characteristics have a high correlation to the flow field. The best first characteristic is the liquid intrinsic characteristic, and the second characteristic is the flow channel interface characteristic.

In the present embodiment, the Reynolds number equation in a tube wall may be used to know the flow field condition of the sample, and the equation is as follows:

$$\mathrm{Re} = \frac{\rho V D}{\mu} = \frac{V D}{\gamma} = \frac{Q D}{\gamma A}.$$

In particular, V is average flow velocity (m/s); D is reaction area tube diameter (m); $\mu$ is hydrodynamic viscosity (Pa s or N·s/m$^2$); $\gamma$ is kinematic viscosity ($\mu/\rho$) (m$^2$/s); $\rho$ is fluid density (kg/m$^3$); Q is volume flow (m$^3$/s); A is cross-sectional area (m$^2$). In particular, V, $\mu$, $\gamma$, and $\rho$ belong to the liquid intrinsic characteristic, and D, Q, and A belong to the flow channel interface characteristic. Therefore, whether the flow field is abnormal may be determined by comparing two of the physical characteristics to the Reynolds number or other relative physical characteristics. In the present embodiment, the reaction area tube diameter may be regarded as the diameter of the reaction area 140 of the sample, but is not limited thereto. It should be noted that the present application does not limit the relationship between the physical characteristics represented by the two physical characteristics, only that the two are do not have the same physical meaning and that both of the two characteristics have a high degree of correlation with the flow field, and most preferably at least one of the characteristics is the liquid intrinsic characteristic; and more preferably at least one liquid intrinsic characteristic and one flow channel interface characteristic.

The following describes how to use the two characteristics (i.e., the first characteristic and the second characteristic) to determine the flow field condition via a first method, a second method, and a third method respectively.

**[First method] Taking the flow velocity of the sample as the first characteristic and the viscosity of the sample as the second characteristic as an example to illustrate how to determine flow field condition using flow velocity and viscosity:**

First, the sample is injected into the reaction area 140 of the electrochemical test strip 100 so that after the sample is injected, the sample may flow into the reaction area 140 via capillary force and continue to flow in the direction of a vent 161. Then, while the sample is flowing, the flow velocity and viscosity of the sample are measured. In the present embodiment, the flow velocity is measured and calculated by measuring the time needed for the sample to pass via the electrode group 121 and the electrode group 122, but is not limited thereto. Specifically, in the present embodiment, the step of measuring the flow velocity of the sample flowing from the electrode group 121 to the electrode group 122 in the reaction area 140 may include: first, the sample flows through and covers the electrode group 121 in the reaction area 140, so that the electrode group 121 may generate the first signal at the first time and record the first time; then, the sample continues to flow through and covers the electrode group 122 in the reaction area 140, so that the electrode group 122 may generate a second signal at the second time and record the second time; then, the difference between the first time and the second time is calculated to obtain the flow velocity. In particular, when the first signal is generated, the sample entered the reaction area 140 and reached the electrode group 121; when the second signal is generated, the sample reached the electrode group 122. In particular, the type of the first signal or the second signal may be, for example, impedance change, capacitive reactance change, voltage change, current change, or a combination of the above on the electrode, but is not limited thereto.

More specifically, since the insulating substrate 110, the first insulating spacer 130, the reaction area 140, and the second insulating spacer 160 are combined to define a single-flow direction capillary, after the sample is injected into the reaction area 140, the sample flows toward the vent 161 due to capillary force, and then flows through and covers the electrode group 121 and the electrode group 122 in sequence. Therefore, the difference between the first time of the first signal and the second time of the second signal is the time for the sample to flow from the electrode group 121 to the electrode group 122 in the reaction area 140. In addition, since a first distance D1 between the electrode group 121 and the electrode group 122 in the reaction area 140 is a fixed value, the difference between the first time and the second time may be regarded as the flow velocity. In particular, the larger the difference, the slower the flow velocity of the sample; conversely, the smaller the difference, the faster the flow velocity of the sample.

In the present embodiment, the viscosity of the sample may be measured in the following way. For example, in an embodiment, since the constituting components and component concentration of the sample itself vary with different samples, different samples have different intrinsic electrical characteristics. Therefore, the ion diffusion and/or migration state of the sample may be known by detecting the intrinsic electrical characteristics of the sample. For example, when the sample is blood, since the blood contains blood cells and plasma, the proportion of human blood cells is between 20% and 65%. Therefore, as the proportion of blood cells is increased, the ion diffusion and/or migration ability of the blood is also changed. Therefore, the intrinsic electrical characteristic of blood, such as the viscosity of blood, may be expressed by detecting the ratio of blood cells in the blood, but is not limited thereto. In some embodiments, the intrinsic electrical characteristic of blood may be obtained via a method such as capacitance, reactance, and diffusion current. In addition, in addition to the ratio of blood cells, the plasma proteins, minerals, or vitamins and the like in the plasma may also affect the ion diffusion and/or migration ability of the blood. In another embodiment, when the sample covers the electrode group 121 and/or the electrode group 122 to change the electrical properties of the electrodes, the measuring instrument 200 may detect the physical characteristics of the sample via the electrode group 121 and/or the electrode group 122 and calculate the viscosity, that is, the flow velocity and the viscosity are measured in the same step.

Then, after the flow velocity and viscosity are measured as described above, the ratio of flow velocity to viscosity is calculated, to determine whether the flow field condition of the sample filled in the reaction area is normal, and to determine whether to display the result of the analysis of the sample. When the ratio is within the predetermined range, the flow field condition of the sample may be determined as normal, and therefore, the result of the analysis of the sample may be displayed. When the ratio is within the correctable range, the concentration measurement result may be corrected via the ratio. Therefore, the concentration measurement result of analysis and correction may also be displayed after correction. Conversely, when the ratio exceeds the predetermined range or correctable range, the flow field condition of the sample may be determined as abnormal. Therefore, "error" may be displayed and the concentration measurement result of analysis of the sample is not displayed. So far, how to determine the flow field condition using the flow velocity of the sample and the liquid electrical characteristic of the sample is clearly explained.

It should be mentioned that, although the intrinsic electrical characteristic of the sample used in the present embodiment is viscosity, and the flow field condition of the sample may be obtained after analyzing the viscosity and flow velocity, the invention does not limit the intrinsic electrical characteristic of the sample to viscosity. In other words, in another embodiment, the intrinsic electrical characteristic of the sample should include at least one of all the physical characteristics changing the flow field, and the physical characteristics may be detected by the electrochemical detection system disclosed in the invention. More preferably, the intrinsic electrical characteristic of the sample may include, for example, signals generated by the physical characteristics of a plurality of samples.

**[Second method] Taking the flow velocity of the sample as the first characteristic, and the empirical liquid electrical characteristic of the sample as the second characteristic as an example to illustrate how to determine flow field condition using flow velocity and liquid electrical characteristic:**

In the present embodiment, since the method of measuring the flow velocity of the sample is similar to the first method, it is not repeated herein. The empirical liquid electrical characteristic of the sample may be obtained according to the correspondence table of the viscosity of the sample and the liquid electrical characteristic after the viscosity of the sample is measured. For example, when the sample is blood, since the viscosity of blood or the blood cell ratio affects the flow velocity of the sample, a correspondence table of blood viscosity (or blood cell ratio) and liquid electrical characteristic may be established by measuring various different blood viscosity (or blood cell ratio) and the corresponding liquid electrical characteristic thereof. In particular, the correspondence table may indicate the empirical liquid electrical characteristic of the blood when the blood is at a specific viscosity (or blood cell ratio). Then, by calculating the ratio of the flow velocity to the liquid electrical characteristic, whether the flow field condition of the sample filled in the reaction area is normal is determined, and whether to display the result of the analysis of the sample is determined. So far, how to determine the flow field condition using the flow velocity of the sample and the empirical liquid electrical characteristic of the sample is clearly explained.

**[Third method] Taking the viscosity of the sample as the first characteristic and the contact angle of the sample to the electrochemical test strip as the second characteristic as an example to illustrate how to determine flow field condition using viscosity and contact angle:**

In the present embodiment, since the method of measuring the viscosity of the sample is similar to the first method, it is not repeated herein. In addition, since the contact angle and resistance have a positive correlation, the contact angle may be calculated by measuring the signal of the blood sample in the flow channel. Then, by calculating the ratio of the viscosity to the contact angle, whether the flow field condition of the sample filled in the reaction area is normal is determined, and whether to display the result of the analysis of the sample is determined. So far, how to determine the flow field condition using the viscosity and contact angle of the sample is clearly explained.

It should be mentioned that, although the intrinsic electrical characteristic of the sample used in the present embodiment is viscosity, and the flow field condition of the sample may be obtained after analyzing the viscosity and contact angle, the invention does not limit the intrinsic electrical characteristic of the sample to viscosity. In other words, in other embodiments, the intrinsic electrical characteristic of the sample may be flow velocity, and in another embodiment, the intrinsic electrical characteristic of the sample should include at least one of all the physical characteristics changing the flow field, and the physical characteristics may be detected by the electrochemical detection system disclosed in the invention. More preferably, the intrinsic electrical characteristic of the sample may include, for example, signals generated by the physical characteristics of a plurality of samples.

It should be mentioned that, although the first method, the second method, and the third method determine the flow field condition using two characteristics as examples, the invention is not limited to determining the flow field condition using only two characteristics. That is to say, in another embodiment, the flow field condition may also be determined using three or more than three characteristics. For example, the temperature may be measured first using the temperature sensor on the measuring instrument, and then three-dimensional analysis may be performed using the temperature characteristic, viscosity, and contact angle to obtain an accurate flow field condition in the reaction area. In addition, the invention does not limit the three-dimensional analysis method. That is to say, in another embodiment, the viscosity may be corrected by temperature first, and then the flow field may be analyzed with the corrected viscosity and flow velocity. In yet another embodiment, the flow velocity, viscosity, and contact angle may be calculated at the same time to analyze the flow field, and then the flow field resolution may be improved via three-dimensional information.

The detailed features of the electrochemical test strip 100 are described in detail below, wherein the detailed features may be applied to all the above embodiments. Referring to FIG. 1B and FIG. 1C again, in the present embodiment, the reaction area 140 has a first side 141 and a second side 142 opposite to the first side 141. The measuring instrument 200 may be electrically connected to the electrode group 121 and the electrode group 122.

Specifically, the reaction area 140 includes a portion 121a of the electrode group 121 and a portion 122a of the electrode group 122. The portion 121a of the electrode group 121 is adjacent to the first side 141 of the reaction area 140, and the portion 122a of the electrode group 122 is adjacent to the second side 142 of the reaction area 140. In the present embodiment, there is the first distance D1 between the portion 121a of the electrode group 121 and the portion 122a of the electrode group 122, and there is a second distance D2 (that is, the length of the reaction area 140 or the concave structure 131) between the first side 141 and the second side 142 of the reaction area 140. The first distance D1 is, for example, the distance between the side of the electrode group 121 adjacent to the first side 141 and the electrode 1221 of the electrode group 122 adjacent to the first side 141 in the reaction area 140. In the present embodiment, the first distance D1 may be, for example, greater than or equal to 0.3 times the second distance D2 (i.e., $D1 \geqq 30\% \times D2$); preferably, the first distance D1 may also be, for example, greater than or equal to 0.5 times the second distance D2 (that is, $D1 \geqq 50\% \times D2$); and more preferably, the first distance D1 may also be, for example, greater than or equal to 0.8 times the second distance D2 (that is, $D1 \geqq 80\% \times D2$), so as to ensure the accuracy of the measured flow velocity.

In the present embodiment, the second insulating spacer 160 covers at least a portion of the reaction area 140 and exposes the connection area 112 of the insulating substrate 110 and the electrode groups 121 and 122 located on the connection area 112.

Hereinafter, the technical means adopted by the invention to achieve the objectives are explained with figures and experimental examples. The following sample is exemplified by blood, but is not limited thereto. Therefore, those ordinary knowledge in the art may readily replace the sample with other liquid samples based on the content disclosed in the present embodiment.

**Experimental examples**

**Experimental example 1: Effect of blood cell ratio on viscosity**

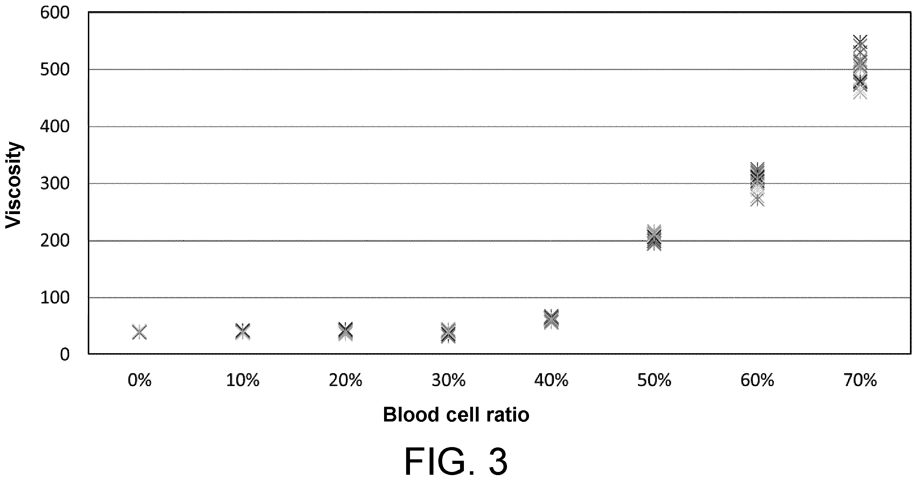

Since the viscosity may be used as a characteristic of the liquid intrinsic characteristic, the following describes how to use the electrochemical detection system shown in FIG. 1 to measure the viscosity of blood. In Experimental Example 1, the blood cell ratio and viscosity of the blood may be detected via the first signal generated by the electrode group 121 at the first time or/and the second signal generated by the electrode group 122 at the second time. Please refer to FIG. 3, the X-axis is the hematocrit (HCT) of different blood samples, and the Y-axis is the viscosity after measurement. It may be seen from the results in FIG. 3 that the viscosity of blood is not changed much in blood with a blood cell ratio of 0% to 30%. However, in blood with a blood cell ratio of 30% to 70%, as the blood cell ratio is increased, the viscosity of the blood is also gradually increased. In particular, in blood with 50% hematocrit ratio, the viscosity of blood is significantly fluctuated or changed (i.e., $200 \pm 20$); and in blood with 70% hematocrit ratio, the viscosity of blood is fluctuated or changed more significantly (i.e., $500 \pm 80$ ms). However, although the viscosity of blood with a blood cell ratio of 50% to 70% has a certain fluctuation range, the viscosity range of blood with various blood cell ratios may still be clearly distinguished. In particular, the fluctuation and the change may be caused by the superimposed effects of protein concentration, red blood cell deformability, and aggregation and the like.

**Experimental Example 2: Compare flow field condition of blood under normal condition and during secondary sample feeding**

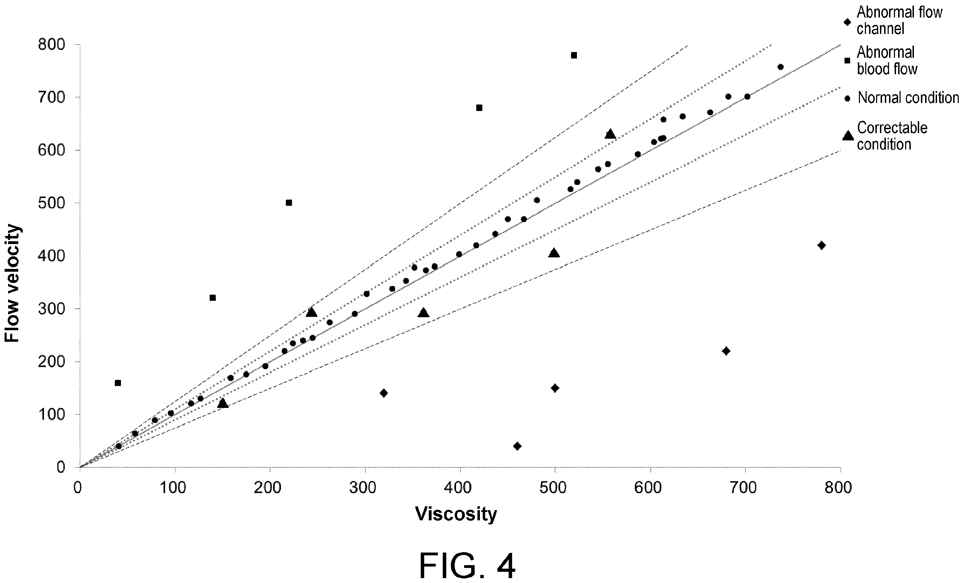

In Experimental Example 2, by using the electrochemical detection system and the electrochemical detection method shown in FIG. 1 to compare the flow field condition of blood under normal condition and during secondary sample feeding, the occurrence of secondary sample feeding may be identified according to the abnormal flow field condition. Specifically, referring to FIG. 4, the flow velocity (Y-axis) and viscosity (X-axis) of 45 blood samples under normal condition (indicated by circular symbols in the figure) and 5 blood samples under abnormal sampling condition (indicated by square symbols in the figure) are measured. In particular, the condition of abnormal sampling may be, for example, secondary sample feeding, but is not limited thereto. The secondary sample feeding refers to the situation where the user suddenly discovers that the blood in the reaction area is insufficient after the blood is injected normally, and then injects more blood again. However, the secondary sample feeding situation destroys the dissolution and diffusion conditions of the reaction layer, thus disrupting the original standing time flow and affecting the measurement result. In addition, this secondary sample feeding situation also changes the original capillary force in the capillary, thus affecting the original flow velocity.

As shown in FIG. 4, the solid line in the figure is the ratio under ideal condition, which means that the flow velocity is proportional to the viscosity, and the ratio of the flow velocity to the viscosity (i.e., $=/$) is equal to 1. The dotted line in the figure indicates that the ratio is still within $\pm 2\%$ of the tolerance. The dashed line in the figure indicates that the ratio is still within $\pm 5\%$ of the correctable error range, so that the blood measurement result in this range may still be corrected via calculation formulas or other correction methods. In the present embodiment, the predetermined range or correctable range of the ratio is, for example, in the range of 0.5 to 1.5 and does not include 0.5 and 1.5 (i.e., 0.5<predetermined range or correctable range<1.5), but is not limited thereto. Preferably, the predetermined range or correctable range is, for example, in the range of 0.8 to 1.2 and does not include 0.8 and 1.2 (i.e., 0.8<predetermined range or correctable range<1.2). More preferably, the predetermined range or correctable range is, for example, in the range of 0.95 to 1.05 and does not include 0.95 and 1.05 (i.e., 0.95<predetermined range or correctable range<1.05). In addition, it should be noted that those with ordinary knowledge in the art may also define the predetermined range or correctable range of the ratio by themselves according to the tolerance of their system.

It may be known from the results of FIG. 4 that, under normal condition, the ratios of the flow velocity to the viscosity of the 45 blood samples are all within a tolerance of $\pm 2\%$. Therefore, the system determines that the flow field conditions of these 45 blood samples are normal, and shows the results of analysis of the 45 blood samples. However, since the flow velocities of the 5 blood samples with abnormal sampling (i.e., secondary sample feeding) are all much greater than the viscosity, the ratios of the flow velocity to the viscosity thereof all exceed the correctable error range of $\pm 5\%$. Therefore, the system determines that the flow field conditions of the 5 blood samples of secondary sample feeding are abnormal, and displays "error" and does not display the results of analysis of the 5 blood samples of secondary sample feeding. It may be seen that the electrochemical detection system and electrochemical detection method shown in FIG. 1 may indeed be used to identify the occurrence of secondary sample feeding.

**Experimental Example 3: Compare flow field condition of blood under normal condition and during secondary use**

In Experimental Example 3, by using the electrochemical detection system and the electrochemical detection method shown in FIG. 1 to compare the flow field condition of blood under normal condition and during the secondary use, the occurrence of secondary use may be identified according to the abnormal flow field condition. Specifically, referring to FIG. 4, the flow velocity (Y-axis) and viscosity (X-axis) of 45 blood samples under normal condition (indicated by circular symbols in the figure) and 5 blood samples under abnormal sampling condition (indicated by square symbols in the figure) are measured. In particular, the abnormal sampling condition may be, for example, secondary use, but is not limited thereto. The secondary use means that when the test strip is used repeatedly, the blood may still enter the reaction area via capillary force and start the electrochemical measurement program under the premise that the vent is not blocked. However, since the reaction layer is dissolved and mixed with the previous blood and crystallized again, the reaction layer in the reaction area at this time presents an unpredictable and irregular state. In addition, the crystallization of the reaction layer and the previous blood also changes the flow channel condition of the reaction area, thus affecting the flow velocity. However, in another embodiment, the crystallization condition of the reaction layer may also be determined based on the sample and electrode interface characteristic.

Since Experimental Example 2 explained the meanings represented by the solid lines, dotted lines, and dashed lines in FIG. 4, and explained the predetermined range or correctable range, they are not repeated herein. It may be known from the results of FIG. 4 that, under normal condition, the ratios of the flow velocity to the viscosity of the 45 blood samples are all within a tolerance of ±2%. Therefore, the system determines that the flow field conditions of these 45 blood samples are normal, and shows the results of analysis of the 45 blood samples. However, since the flow velocities of the 5 blood samples with abnormal sampling (i.e., secondary use) are all much greater than the viscosity, the ratios of the flow velocity to the viscosity thereof all exceed the correctable error range of ±5%. Therefore, the system determines that the flow field conditions of the 5 blood samples of secondary use are abnormal, and displays "error" and does not display the results of analysis of the 5 blood samples of secondary use. It may be seen that the electrochemical detection system and electrochemical detection method shown in FIG. 1 may indeed be used to identify the occurrence of secondary use.

**Experimental Example 4: Compare flow field condition of blood under normal condition and during abnormal flow channel**

In Experimental Example 4, by using the electrochemical detection system and the electrochemical detection method shown in FIG. 1 to compare the flow field condition of blood under normal condition and during abnormal flow channel, the occurrence of abnormal flow channel may be identified according to the abnormal flow field condition. Specifically, referring to FIG. 4, the flow velocity (Y-axis) and viscosity (X-axis) of 45 blood samples under normal condition (indicated by circular symbols in the figure) and 5 blood samples under abnormal flow channel condition (indicated by diamond symbols in the figure) are measured. In particular, the abnormal flow channel condition may indicate, for example, that the electrochemical test strip is deformed due to the manufacturing process, is poorly fitted, burrs or foreign objects falling into the reaction area when the concave structure is cut, but is not limited thereto. In addition, the abnormal flow channel situation also changes the flow channel condition in the reaction area, thus affecting flow velocity.

Since Experimental Example 2 explained the meanings represented by the solid lines, dotted lines, and dashed lines in FIG. 4, and explained the predetermined range or correctable range, they are not repeated herein. It may be known from the results of FIG. 4 that, under normal condition, the ratios of the flow velocity to the viscosity of the 45 blood samples are all within a tolerance of ±2%. Therefore, the system determines that the flow field conditions of these 45 blood samples are normal, and shows the results of analysis of the 45 blood samples. However, since the flow velocities of the 5 blood samples with abnormal flow channel are all much smaller than the viscosity, the ratios of the flow velocity to the viscosity thereof all exceed the correctable error range of ±5%. Therefore, the system determines that the flow field conditions of the 5 blood samples of abnormal flow channel are abnormal, and displays "error" and does not display the results of analysis of the 5 blood samples of abnormal flow channel. It may be seen that the electrochemical detection system and electrochemical detection method shown in FIG. 1 may indeed be used to identify the occurrence of abnormal flow channel.

It should be noted that although Experimental Example 4 shows that the flow field condition of abnormal flow channel may be determined and identified using the flow velocity and viscosity of the sample, the invention does not limit the way of determining and identifying abnormal flow channel. In other experimental examples, the occurrence of abnormal flow channel may also be determined and identified using contact angle, as shown in Experimental Example 6.

**Experimental Example 5: Compare flow field condition of blood under normal condition and under correctable condition**

In Experimental Example 5, by using the electrochemical detection system and the electrochemical detection method shown in FIG. 1 to compare the flow field condition of blood under normal condition and under correctable condition, it is identified that the blood has extreme viscosity or the flow channel in the reaction area has a slight defect according to the flow field condition. Specifically, referring to FIG. 4, the flow velocity (Y-axis) and viscosity (X-axis) of 45 blood samples under normal condition (indicated by circular symbols in the figure) and 5 blood samples under correctable condition (indicated by triangle symbols in the figure) are measured. In particular, the correctable condition may be, for example, that the blood sample has extreme viscosity or the flow channel in the reaction area has a slight defect, and the cause and scope of the error may be known via the techniques disclosed in the present application. Therefore, the measuring instrument may perform correction via calculation formulas or other correction methods, but is not limited thereto.

Since Experimental Example 2 explained the meanings represented by the solid lines, dotted lines, and dashed lines in FIG. 4, and explained the predetermined range or correctable range, they are not repeated herein. It may be known from the results of FIG. 4 that, under normal condition, the ratios of the flow velocity to the viscosity of the 45 blood samples are all within a tolerance of ±2%. Therefore, the system determines that the flow field conditions of these 45 blood samples are normal, and shows the results of analysis of the 45 blood samples. However, the flow velocities of the 5 blood samples of correctable condition (that is, the blood sample has extreme viscosity) are slightly larger or slightly smaller than the viscosity, so that the ratios of the flow velocity to the viscosity thereof all fall within the correctable error range of ±5%. Therefore, the system may correct the result thereof via calculation formulas or other correction methods and display the corrected measurement results.

**Experimental Example 6: Compare flow field condition of blood under normal condition and during abnormal flow channel**

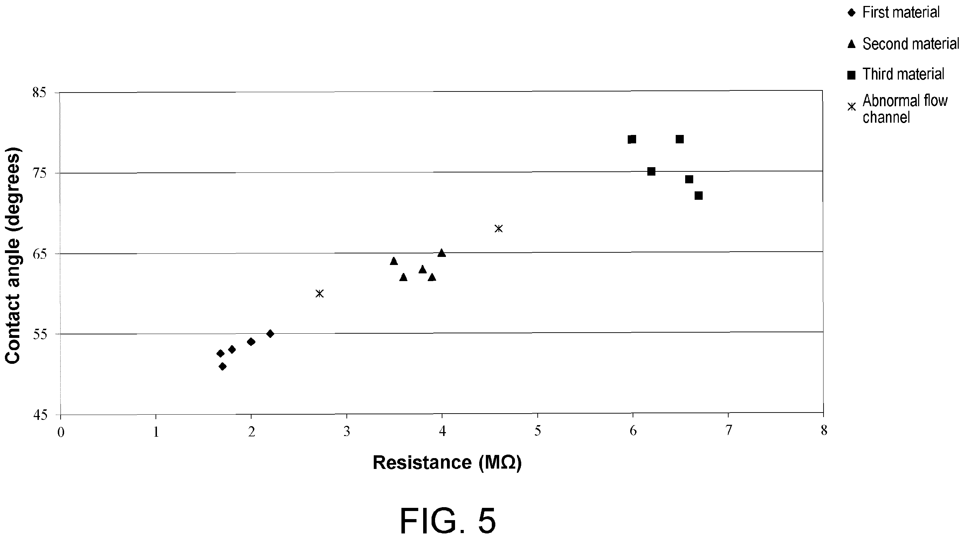

In Experimental Example 6, by using the electrochemical detection system and the electrochemical detection method shown in FIG. 1 to compare the flow field condition of blood under normal condition and during abnormal flow channel, the occurrence of abnormal flow channel may be identified according to the abnormal flow field condition. In detail, please refer to FIG. 5. In the electrochemical test strips made of three different materials, the contact angle (Y-axis) and resistance (X-axis) of 5 different blood samples under normal condition are detected respectively. In the figure, diamond symbols indicate the detection results of 5 different blood samples at electrochemical test strips made of a first material. In the figure, triangle symbols indicate the detection results of 5 different blood samples at electrochemical test strips made of a second material. In the figure, square symbols indicate the detection results of 5 different blood samples at electrochemical test strips made of a third material. Then, the contact angle and resistance of two different blood samples under abnormal flow channel condition (indicated by * in the figure) are respectively detected. In particular, since the contact angle and resistance have a positive correlation, the contact angle may be calculated by measuring the signal of the blood samples in the flow channel.

It may be known from the results of FIG. 5 that, although the contact angle and resistance in the electrochemical test strips made of the first material, the second material, and the third material are all different for the 5 different blood samples, the difference in contact angle and resistance caused by the three materials is more significant. In other words, the difference in contact angle caused by different materials is more significant than the difference in contact angle caused by different blood samples. Therefore, the contact angle may be used as the sample and electrode interface characteristic.

Next, when the potential contact angle range and resistance range of the blood sample are defined using the contact angle and resistance of 5 different blood samples under normal condition, it may be found that the contact angle and resistance of two different blood samples under abnormal flow channel condition are all outside the range. Therefore, the system may determine the abnormal flow field conditions of the blood samples with abnormal flow channel based on the relationship between contact angle and resistance, and displays "error" and not display the results of analysis of the two blood samples with abnormal flow channel. It may be seen that the electrochemical detection system and electrochemical detection method shown in FIG. 1 may indeed be used to identify the occurrence of abnormal flow channel.

**Experimental Example 7: Measuring equivalent relationship between contact angle and viscosity**

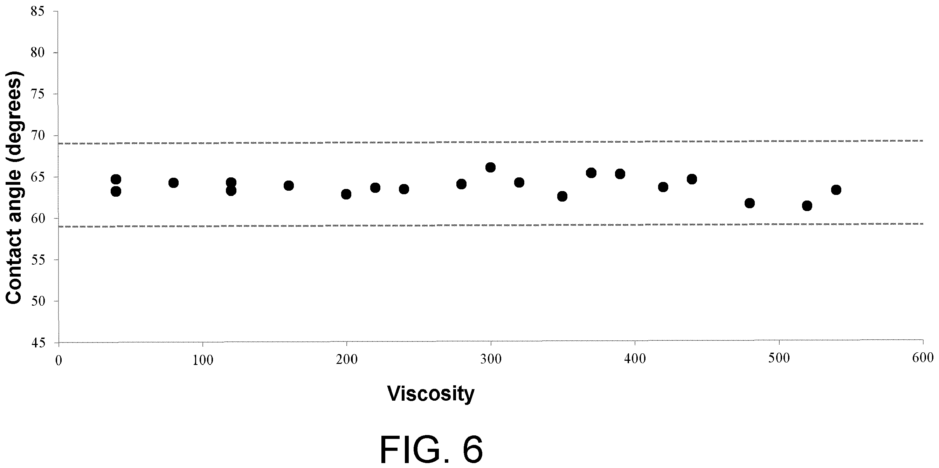

In Experimental Example 7, the equivalent relationship between contact angle and viscosity is measured by using the electrochemical detection system and electrochemical detection method shown in FIG. 1 to ensure that the contact angle and viscosity may be used as the first characteristic and the second characteristic for determining the flow field condition. In detail, please refer to FIG. 6, the contact angle and viscosity of 20 different blood samples with different blood cell ratios are measured using the electrochemical test strip made of the second material in Experimental Example 6.

It may be seen from the results in FIG. 6 that the viscosity of 20 blood samples with different blood cell ratios may be substantially distributed between about 40 and 550, and the contact angle thereof may be substantially distributed between about 60 degrees and 70 degrees. That is to say, even though the viscosity difference between 20 blood samples with different blood cell ratios is quite large, the difference in contact angle between the 20 blood samples with different blood cell ratios is not significant. Therefore, there is a low equivalence relationship between viscosity and contact angle. Moreover, since the viscosity may be used as the liquid intrinsic characteristic of the blood sample, and the contact angle may be used as the blood sample and electrode interface characteristic, in other experimental examples, the flow field conditions of blood samples may be determined using the relationship between viscosity and contact angle.

**Experimental example 8: Compare measurement deviation value of detection method of electrochemical detection system shown in FIG. 1 with detection method of general electrochemical detection system**

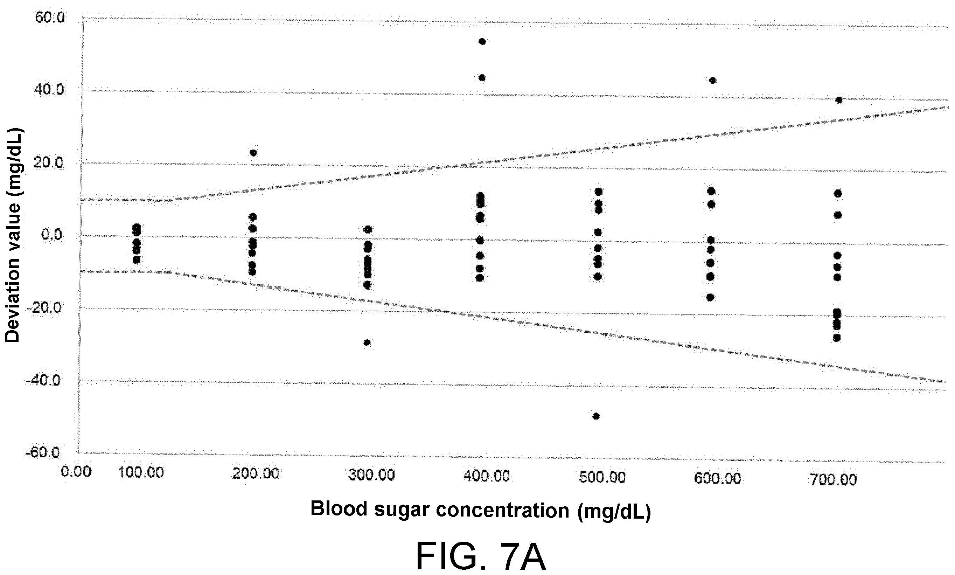

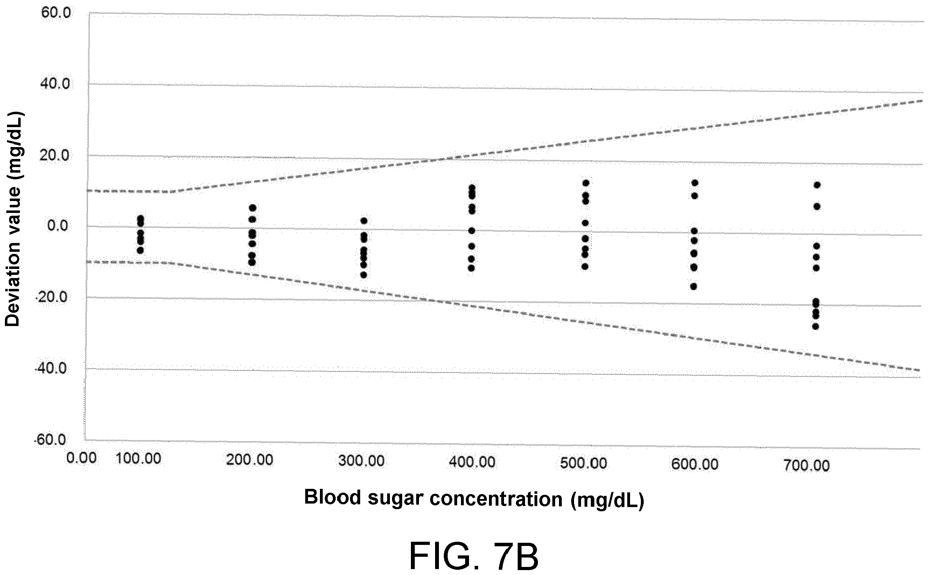

In Experimental Example 7, the flow field condition of the sample is determined using the electrochemical detection system and electrochemical detection method shown in FIG. 1 in order to correct or eliminate the issue of errors in the measurement results of the sample concentration caused by abnormal flow field condition, thereby improving detection accuracy. Specifically, referring to FIG. 7A and FIG. 7B, in FIG. 7A, the blood sugar concentration of different blood samples is measured (100, 200, 300, 400, 500, 600, 700 mg/dL) using a general electrochemical test strip detection method, and in FIG. 7B, the blood sugar concentration of different blood samples is measured (100, 200, 300, 400, 500, 600, 700 mg/dL) using the electrochemical test strip detection method of the present embodiment; then, the measurement results of the two electrochemical test strips are compared with the measurement results of the large measuring instrument YSI to obtain the deviation value. In particular, the X-axis is the blood sugar concentration, and the Y-axis is the deviation value between the result measured by the electrochemical test strip and the result measured by the large measuring instrument YSI. In addition, the dashed lines in the figure represent the allowable error range.

It may be known from the results of FIG, 7A that, in the measurement results of the general electrochemical test strip detection method, most of the deviation values after comparison with the large measuring instrument YSI are within the dashed lines (that is, within the allowable error range), but there are still about 10% of the deviation values outside the dashed lines (that is, outside the allowable error range), indicating that about 10% of the measurement results have a significant error with the general electrochemical test strip detection method. Conversely, it may be seen from the results of FIG. 7B that, in the measurement results of the detection method using the electrochemical test strip of the present embodiment, since during the measurement of the electrochemical test strip of the present embodiment, an abnormal condition may be determined, corrected, or eliminated via the ratio of flow velocity to viscosity, the deviation values of all the results after comparison with the results of the large measuring instrument YSI are within the dashed lines (that is, within the allowable error range). That is to say, compared with the detection method using a general electrochemical test strip, the detection method of the electrochemical test strip of the present embodiment may significantly improve detection accuracy.

Based on the above, in the electrochemical detection system and the electrochemical detection method of an embodiment of the invention, by measuring the first characteristic and the second characteristic, and calculating the ratio of the first characteristic to the second characteristic, whether the flow field condition of the sample is normal may be determined. In particular, only when the ratio is within a predetermined range is the flow field condition determined as normal and a result of an analysis of the sample displayed. In addition, in the present embodiment, the result may be corrected or the result of the abnormal condition may be eliminated according to the ratio. In this way, the electrochemical detection system and the electrochemical detection method of the present embodiment may avoid errors, or may have the effect of improving detection accuracy.

## Claims

1. An electrochemical detection system (10), configured to detect a sample, comprising:

   an electrochemical test strip (100) comprising a main body (101) and at least one electrode group (121, 122); and
   a measuring instrument (200) electrically connected to the at least one electrode group (121, 122) and adapted to determine whether a flow field condition of the sample is normal via the at least one electrode group (121, 122).

2. The electrochemical detection system (10) of claim 1, wherein the measuring instrument (200) measures a first characteristic and a second characteristic of at least one of the sample and the electrochemical test strip (100) via the at least one electrode group (121, 122), and calculates a ratio of the first characteristic to the second characteristic to determine whether a flow field condition of the sample is normal, wherein when the ratio is within a predetermined range, the measuring instrument (200) determines that the flow field condition is normal and displays a result of an analysis of the sample.

3. The electrochemical detection system (10) of claim 2, wherein the first characteristic or the second characteristic is a fluid characteristic.

4. The electrochemical detection system (10) of claim 3, wherein the fluid characteristic comprises a flow velocity, a temperature, a viscosity, and a density of the sample.

5. The electrochemical detection system (10) of claim 2, wherein the first characteristic or the second characteristic is a flow channel characteristic.

6. The electrochemical detection system (10) of claim 5, wherein the flow channel characteristic comprises a contact angle, a charging current, a diameter of a reaction area of the sample, a volume flow rate of the sample, and a cross-sectional area of the reaction area.

7. The electrochemical detection system (10) of claim 2, wherein there is a low equivalence between the first characteristic and the second characteristic.

8. The electrochemical detection system (10) of claim 1, wherein the flow field condition comprises an abnormal flow channel and an abnormal sampling.

9. A measuring instrument (200), configured to detect a sample, comprising:

   a connector (210);
   a microcontroller (220) electrically connected to at least one electrode group (121, 122) via the connector (210);
   a power module (230) electrically connected to the microcontroller (220); and
   a display (250) electrically connected to the microcontroller (220),
   wherein the microcontroller (220) is adapted to measure a first characteristic and a second characteristic of the sample via the at least one electrode group (121, 122), and analyze a relationship between the first characteristic and the second characteristic and a flow field.

**10.** An electrochemical detection method, configured to detect a sample, comprising:

> injecting the sample into an electrochemical test strip (100); and
> determining whether a flow field condition of the sample is normal via a measuring instrument (200),
> wherein the step of determining whether the flow field condition of the sample is normal comprises:
>
>> measuring a first characteristic and a second characteristic of at least one of the sample and the electrochemical test strip (100) via at least one electrode group (121, 122) of the electrochemical test strip (100); and
>> calculating a ratio of the first characteristic to the second characteristic, wherein when the ratio is within a predetermined range, the measuring instrument (200) determines that the flow field condition is normal and displays a result of an analysis of the sample.

**11.** The electrochemical detection method of claim 10, wherein the first characteristic is related to an ion diffusion and/or a migration ability of the sample.

**12.** The electrochemical detection method of claim 11, wherein the first characteristic comprises a flow velocity, a temperature, a viscosity, and a density of the sample.

**13.** The electrochemical detection method of claim 10, wherein the second characteristic is related to a sample and electrode interface characteristic.

**14.** The electrochemical detection method of claim 13, wherein the second characteristic comprises a contact angle, a charging current, a diameter of a reaction area of the sample, a volume flow rate of the sample, and a cross-sectional area of the reaction area.

**15.** The electrochemical detection method of claim 10, wherein there is a low equivalence between the first characteristic and the second characteristic.

FIG. 1A

FIG. 1B

FIG. 1C

S1

Inject a sample into a reaction area of an electrochemical test strip

S2

Measure a first parameter and a second parameter of the sample

S3

A flow field condition is determined as normal when a ratio is in a predetermined range, and display a result of an analysis of the sample

FIG. 2

FIG. 3

EP 4 124 854 A1

FIG. 4

FIG. 5

EP 4 124 854 A1

FIG. 6

EP 4 124 854 A1

FIG. 7A

**FIG. 7B**

EP 4 124 854 A1

Europäisches
Patentamt

European
Patent Office

Office européen
des brevets

# EUROPEAN SEARCH REPORT

Application Number

EP 22 15 2850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/082575 A1 (TSAI TSUNG-HSUAN [TW] ET AL) 23 March 2017 (2017-03-23) * paragraphs [0010] - [0032], [0044] - [0060]; figures 1-8 * | 1-15 | INV. G01N27/327 |
| X | US 2003/098233 A1 (KERMANI MAHYAR Z [US] ET AL) 29 May 2003 (2003-05-29) * paragraphs [0052] - [0074]; figures 1-8 * | 1-15 | |
| X | WO 2005/078437 A1 (BAYER HEALTHCARE LLC [US]; HUANG DIJIA [US] ET AL.) 25 August 2005 (2005-08-25) * paragraphs [0017] - [0034]; figures 1-4 * | 1-15 | |
| X | EP 2 239 563 A1 (BIONIME CORP [TW]) 13 October 2010 (2010-10-13) * paragraphs [0073] - [0084]; figures 1-11 * | 1-15 | |
| A | US 5 352 351 A (WHITE BRADLEY E [US] ET AL) 4 October 1994 (1994-10-04) * the whole document * | 1-15 | TECHNICAL FIELDS SEARCHED (IPC) G01N |
| A | US 2016/131610 A1 (CHU CHING YUAN [TW] ET AL) 12 May 2016 (2016-05-12) * the whole document * | 1-15 | |

The present search report has been drawn up for all claims

| Place of search | Date of completion of the search | Examiner |
|---|---|---|
| Munich | 23 June 2022 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 2850

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2022

| Patent document cited in search report | Publication date | Patent family member(s) | Publication date |
|---|---|---|---|
| US 2017082575 A1 | 23-03-2017 | TW 201712329 A | 01-04-2017 |
| | | US 2017082575 A1 | 23-03-2017 |
| US 2003098233 A1 | 29-05-2003 | CA 2407249 A1 | 10-04-2003 |
| | | CA 2838176 A1 | 10-04-2003 |
| | | CN 1412548 A | 23-04-2003 |
| | | CN 101311723 A | 26-11-2008 |
| | | CN 102621211 A | 01-08-2012 |
| | | CZ 20023368 A3 | 18-06-2003 |
| | | HK 1055463 A1 | 09-01-2004 |
| | | JP 4354168 B2 | 28-10-2009 |
| | | JP 2003185615 A | 03-07-2003 |
| | | KR 20030030955 A | 18-04-2003 |
| | | MX PA02010106 A | 14-10-2004 |
| | | PL 356578 A1 | 22-04-2003 |
| | | RU 2292841 C2 | 10-02-2007 |
| | | TW 581866 B | 01-04-2004 |
| | | US 2003098233 A1 | 29-05-2003 |
| | | US 2005023154 A1 | 03-02-2005 |
| WO 2005078437 A1 | 25-08-2005 | AT 532063 T | 15-11-2011 |
| | | BR PI0507322 A | 26-06-2007 |
| | | CA 2554060 A1 | 25-08-2005 |
| | | CN 1918471 A | 21-02-2007 |
| | | EP 1714148 A1 | 25-10-2006 |
| | | JP 4814110 B2 | 16-11-2011 |
| | | JP 2007523327 A | 16-08-2007 |
| | | RU 2371707 C2 | 27-10-2009 |
| | | US 2007045127 A1 | 01-03-2007 |
| | | US 2011061458 A1 | 17-03-2011 |
| | | US 2012132542 A1 | 31-05-2012 |
| | | US 2013161206 A1 | 27-06-2013 |
| | | US 2014202881 A1 | 24-07-2014 |
| | | US 2016274052 A1 | 22-09-2016 |
| | | WO 2005078437 A1 | 25-08-2005 |
| EP 2239563 A1 | 13-10-2010 | EP 2239563 A1 | 13-10-2010 |
| | | TW 201037301 A | 16-10-2010 |
| | | US 2010258453 A1 | 14-10-2010 |
| | | US 2017038330 A1 | 09-02-2017 |
| US 5352351 A | 04-10-1994 | AU 6832594 A | 03-01-1995 |
| | | CA 2153877 A1 | 22-12-1994 |
| | | DE 702788 T1 | 02-11-2000 |
| | | DE 69434438 T2 | 12-01-2006 |
| | | DE 69434836 T2 | 18-10-2007 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 15 2850

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2022

| Patent document cited in search report | Publication date | Patent family member(s) | | Publication date |
|---|---|---|---|---|
| | | EP | 0702788 A1 | 27-03-1996 |
| | | EP | 1582864 A1 | 05-10-2005 |
| | | ES | 2153334 T1 | 01-03-2001 |
| | | ES | 2270401 T3 | 01-04-2007 |
| | | JP | 2800981 B2 | 21-09-1998 |
| | | JP | H08502589 A | 19-03-1996 |
| | | US | 5352351 A | 04-10-1994 |
| | | WO | 9429706 A1 | 22-12-1994 |
| US 2016131610 A1 | 12-05-2016 | CN | 105588862 A | 18-05-2016 |
| | | TW | 201617608 A | 16-05-2016 |
| | | US | 2016131610 A1 | 12-05-2016 |

EPO FORM P0459

For more details about this annex : see Official Journal of the European Patent Office, No. 12/82